# EUROPÄISCHE PATENTSCHRIFT

(11) **EP 0 509 359 B1**
(45) Veröffentlichungstag und Bekanntmachung des Hinweises auf die Patenterteilung: **28.02.1996**
(21) Anmeldenummer: 92105894.7
(22) Anmeldetag: 06.04.1992
(51) Int. Cl.: C07D 215/14, A61K 31/47

(54) **Verfahren zur Herstellung von enantiomerenreinen substituierten (Chinolin-2-yl-methoxy)phenyl-essigsäuren**
Process for the preparation of pure enantiomers of substituted (quinolin-2-yl-methoxy)phenylacetic acids
Procédé pour la préparation d'énantiomères purs d'acides (chinolin-2-yl)méthoxy)phénylacétique substituées

(30) Priorität: 17.04.1991 DE 4112533
(43) Veröffentlichungstag der Anmeldung: 21.10.1992
(73) Patentinhaber: BAYER AG, D-51368 Leverkusen (DE)
(72) Erfinder: Decker, Matthias, Dr., W-5600 Wuppertal 11 (DE); Mohrs, Klaus-Helmut, Dr., W-5600 Wuppertal 1 (DE); Raddatz, Siegfried, Dr., W-5000 Köln 80 (DE)

(56) Entgegenhaltungen:
- EP-A- 0 499 926
- TETRAHEDRON LETTERS. vol. 30, no. 21, 1989, OXFORD GB pages 2825 - 2828; KAORU FUJI ET AL.: 'Binaphtol as a chiral auxiliary. Asymmetric alkylation of arylacetic acid.'
- TETRAHEDRON LETTERS. vol. 30, no. 3, 1989, OXFORD GB pages 327 - 330; A. GUY ET AL.: 'Stereoselective acetoxylation of chiral phenylacetic esters'
- JOURNAL OF THE CHEMICAL SOCIETY, CHEMICAL COMMUNICATIONS. 1987, LETCHWORTH GB pages 656 - 661; AKIRO ANDO ET AL.: 'Asymmetric synthesis using chiral bases: enantioselective alpha-alkylation of carboxylic acids'

## Beschreibung

Die Erfindung betrifft ein Verfahren zur Herstellung von teilweise bekannten enantiomerenreinen (Chinolin-2-yl- methoxy)phenyl-essigsäuren, die als Hemmer von enzymatischen Reaktionen im Rahmen des Arachidonsäurestoffwechsels, insbesondere der Lipoxygenase eingesetzt werden.

Aus der Literatur ist bereits bekannt, daß sich chirale 4-substituierte Phenylessigsäureester für stereoselektive Acetoxylierungen eignen und bei der asymmetrisch kontrollierten Oxidation von aromatischen Verbindungen über eine Donor-Acceptor-Interaktion eingesetzt werden [vgl. J. Chem. Soc., Chem. Commun. 1986, S. 741 - 742; Tetrahedron Letters Vol. 30, Nr. 3, S. 327 - 330, 1989].

Außerdem ist die enantioselektive a-Alkylierung von Carbonsäuren mit Hilfe chiraler Basen aus dem Stand der Technik bekannt [J. Chem. Soc. Chem. Commun., 1987, 656-661]. Ebenso wurde die stereoselektive Acetoxylierung von chiralen Phenylessigsäurederivaten beschrieben [Tetrahedron Lett. 30, 2825 (1989)].

Ebenso ist bekannt, daß enantiomerenreine 4-(Chinolin-2-yl-methoxy)phenyl-essigsäuren durch Diastereomerentrennung nach üblichen Methoden, beispielsweise durch Chromatographie oder fraktionierte Kristallisation, in die entsprechenden Enantiomeren getrennt werden können [vgl. DOS 39 16 663].

Dieses Verfahren hat mehrere Nachteile: Sowohl die chromatographische Diastereomerentrennung als auch die fraktionierte Kristallisation der Diastereomeren ist mit einem großen technischen Aufwand verbunden. Außerdem fallen hierbei in der Regel fünfzig Prozent des "falschen" Diastereomeren an, das Dieser fünfzig prozentige Ausbeuteverlust beeinträchtigt die Wirtschaftlichkeit eines (groß)technischen Verfahrens erheblich, ganz abgesehen davon, daß fünfzig Prozent "Nebenprodukt" zu entsorgen sind. Ferner sind die üblichen chiralen Hilfsreagenzien im allgemeinen bereits in kleinen Mengen sehr teuer und können dann meist nur über einen aufwendigen Syntheseweg hergestellt werden.

Es wurde nun gefunden, daß man enantiomerenreine (Chinolin-2-yl-methoxy)phenylessigsäuren der allgemeinen Formel (I) 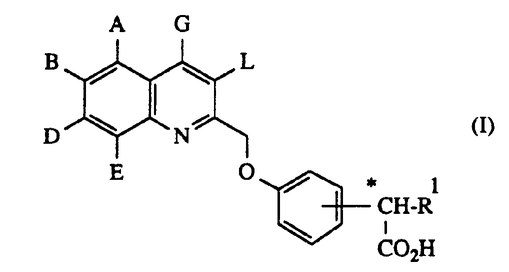 in welcher
A, B, D, E, G und L gleich oder verschieden sind und
   für Wasserstoff, Hydroxy, Halogen, Carboxy, Nitro, Trifluormethyl, Trifluormethoxy oder für geradkettiges oder verzweigtes Alkyl oder Alkoxy mit jeweils bis zu 8 Kohlenstoffatomen stehen, oder
   für Aryl mit 6 bis 10 Kohlenstoffatomen stehen, das gegebenenfalls durch Halogen, Hydroxy, Nitro oder Cyano substituiert ist,

   und
R¹ für Cyclolalkyl mit 4 bis 12 Kohlenstoffatomen seht, erhält, indem man

Verbindungen der allgemeinen Formel (II) 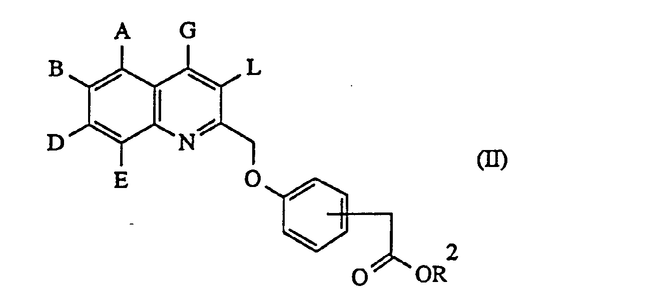 in welcher
A, B, D, E, G und L die oben angegebene Bedeutung haben,
   und
R² die Bedeutung (+)- oder (-)-Menthyl, (+)- oder (-)-Bornyl (+)- oder (-)-Isobornyl oder (-)-8-Phenylmenthyl hat,

zunächst mit Verbindungen der allgemeinen Formel (III) 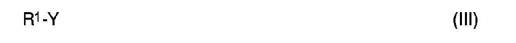 in welcher
R¹ die oben angegebene Bedeutung hat
   und
Y für Brom, Chlor, Jod, Mesyl, Tosyl oder Trifluormethylsulfonyl, vorzugsweise für Jod oder Brom steht,

in inerten Lösemitteln in Anwesenheit einer Base durch diastereoselektive Alkylierung in die Verbindungen der allgemeinen Formel (IV) 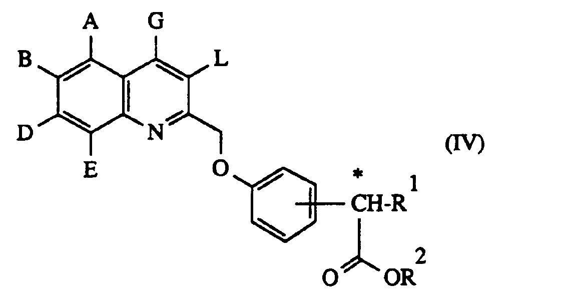 in welcher
A, B, D, E, G, L, R¹ und R² die oben angegebene Bedeutung haben,

überführt
und in einem zweiten Schritt den Rest R² gezielt mit Säuren ohne Racemisierung abspaltet.

Das erfindungsgemäße Verfahren kann durch folgendes Formelschema beispielhaft erläutert werden: 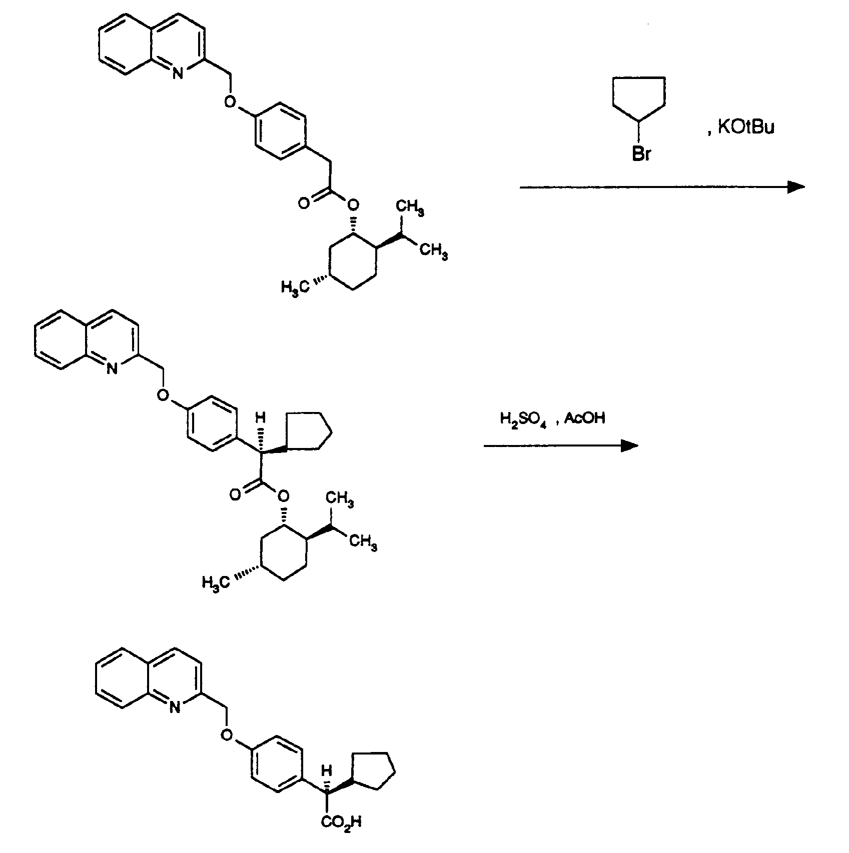 Überraschenderweise liefert das erfindungsgemäße Verfahren die gewünschten enantiomerenreinen (Chinolin-2-yl- methoxy)phenylessigsäuren ohne großen technischen Aufwand in sehr guten Ausbeuten und hoher Reinheit.

Je nach Konfiguration des Restes R² und sterischen Effekten des verwendeten Alkylhalogenids (111) erfolgt die Alkylierung der Verbindung (11) in hohen Ausbeuten und auf einfache Weise erstmals diastereoselektiv. Die Verbindungen (IV) fallen mit hohem Diastereomerenüberschuß an und kristallisieren aus der Reaktionsmischung direkt aus, wodurch bereits die einfache Kristallisation der Rohprodukte die Verbindungen der Formel (IV) in diastereomerenreiner Form liefert.

Ein weiterer Vorteil des erfindungsgemäßen Verfahrens besteht darin, daß durch geeignete Wahl des Lösungsmittels und einer Base das unerwünschte Diastereomer zu dem erwünschten epimerisiert werden kann, welches wiederum direkt auskristallisiert. Somit kann aus den Mutterlaugen durch wiederholte Epimerisierung und Kristallisation weiteres (gewünschtes) diastereomerenreines Produkt gewonnen werden. Durch direktes Zumischung der Mutterlaugen in den Alkylierungsschritt kann das gesamte Verfahren in Form eines Kreisprozesses optimiert werden.

Weiterhin ist ein großer Vorteil des erfindungsgemäßen Verfahrens, daß die Ausgangsverbindungen sehr gut zugänglich sind. Sie lassen sich aus einfacheren Bausteinen unter geringem technischem Aufwand in guten Ausbeuten herstellen. Darüberhinaus ermöglicht das erfindungsgemäße Verfahren, vorhandene Mengen bekannter Racemate der Verbindungen der allgemeinen Formel (I) in die entsprechenden Enantiomere zu überführen. Das erfindungsgemäße Verfahren ermöglicht die Herstellung der erfindungsgemäßen Verbindungen der allgemeinen Formel (I) mit wenigen Synthesestufen und einer wesentlich höheren Gesamtausheute als nach aus dem Stand der Technik bekannten Verfahren.

Die nach dem erfindungsgemäßen Verfahren hergestellten (Chinolin-2-yl-methoxy)phenylessigsäuren sind durch die Formel (I) allgemein definiert. Die entsprechenden Salze können nach üblichen Methoden mit Basen erhalten werden.

Nach dem erfindungsgemäßen Verfahren werden bevorzugt enantiomerenreine (Chinolin-2-yl-methoxy)phenyles- sigsäuren der allgemeinen Formel (I), in welcher
A, B, D, E, G und L gleich oder verschieden sind und für Wasserstoff, Fluor, Chlor, Brom, Trifluormethyl, oder für geradkettiges oder verzweigtes Alkyl mit bis zu 6 Kohlenstoffatomen stehen
   und
R¹ für Cyclopentyl, Cyclohexyl, Cycloheptyl oder Cyclooctyl steht hergestellt.

Nach dem erfindungsgemäßen Verfahren werden besonders bevorzugt (Chinolin-2-yl-methoxy)phenylessigsäuren der allgemeinen Formel (I),
in welcher
A, B, D, E, G und L für Wasserstoff stehen

und
R¹ für Cyclopentyl, Cyclohexyl oder Cycloheptyl steht,

hergestellt.

Ganz besonders bevorzugt werden nach dem erfindungsgemäßen Verfahren enantiomeren reine (Chinolin-2-yl- methoxy)phenylessigsäuren der allgemeinen Formel (I) hergestellt,
in denen
A, B, D, E, G und L für Wasserstoff stehen und der Rest CH-R¹⁻CO_{z}H in 4-Stellung zum Chinolylmethoxyrest steht.

Als Lösemittel für die Alkylierung eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Dimethylformamid.

Die Alkylierung wird in den oben aufgeführten Lösemitteln, gegebenenfalls unter Schutzgasatmosphäre, bei Temperaturen von -20°C bis +100°C, vorzugsweise bei -10°C bis +30°C bei Normaldruck durchgeführt

Als Basen für die diastereoselektive Alkylierung eignen sich die üblichen basischen Verbindungen. Hierzu gehören Alkalihydride wie Natriumhydrid, Alkaliamide wie Natriumamid, Alkalialkoholate wie Natriummethanolat, Natriumethanolat, Kaliummethanolat, Kaliumethanolat oder Kalium-tert.butylat oder organische Amine wie Trialkylamine z.B. Triethylamin, oder lithiumorganische Verbindungen wie Butyllithium oder Phenyllithium. Bevorzugt ist Kalium-tert.butylat.

Bei der diastereoselektiven Alkylierung wird die Base in einer Menge von 1 mol bis 10 mol, bevorzugt von 1,2 mol bis 3 mol bezogen auf 1 mol der Verbindungen der allgemeinen Formel (11) eingesetzt.

Für die Abspaltung des chiralen Restes R² eignen sich die üblichen organischen Carbonsäuren, wie beispielsweise Essigsäure, Ameisensäure, oder anorganische Säuren wie beispielsweise Bromwasserstoffsäure, Chlorwasserstoffsäure oder Schwefelsäure oder Gemische der genannten Säuren. Bevorzugt sind Essigsäure, Ameisensäure, Bromwasserstoffsäure und/oder Schwefelsäure. Ganz besonders bevorzugt ist das Gemisch Essigsäure/Schwefelsäure sowie Ameisensäure/Bromwasserstoffsäure und Ameisensäure/Schwefelsäure.

Die Säuren bzw. deren Gemische werden gleichzeitig als Lösemittel verwandt und somit in einem großen Überschuß ß eingesetzt.

Die Abspaltung erfolgt in einem Temperaturbereich von 0°C bis +150°C, bevorzugt von 40°C bis 100°C.

Sie kann im allgemeinen bei Normaldruck, gegebenenfalls aber auch bei Über-oder Unterdruck (z. B. 0,5 bis 3 bar) durchgeführt werden.

Nach Neutralisation mit Basen in Wasser oder in einem der oben aufgeführten Lösemittel, insbesondere in einem WasserlToluol-Gemisch werden die Säuren nach üblicher Methode aufgearbeitet.

Als Basen zur Neutralisation eignen sich Alkalihydroxide wie Natriumhydroxid oder Kaliumhydroxid. Bevorzugt ist Natriumhydroxid.

Die enantiomerenreinen Rohprodukte der allgemeinen Formel (I) werden nach üblicher Methode, beispielsweise durch Waschen in einem der oben aufgeführten Lösemittel, vorzugsweise Isopropanol durch Chromatographie über Kieselgel gereinigt.

Die enantiomerenreinen Verbindungen der allgemeinen Formel (I) sind im Prinzip aus DOS 3 916 663 bekannt und steilen wertvolle Wirkstoffe zur Herstellung von Arzneimitteln, insbesondere von Lipoxygenasehemmern dar.

Die Ausgangsverbindungen der Formel (II) sind neu und werden hergestellt, indem man Hydroxyphenylessigsäurederivate der allgemeinen Formel (V) 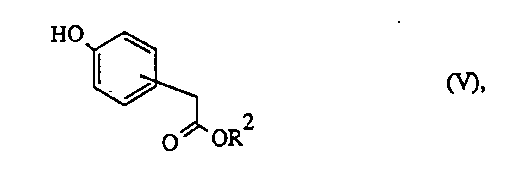 in welcher
R² die Bedeutung (+)- oder (-)-Menthyl, (+)- oder (-)-Bornyl, (+)- oder (-)-Isobornyl oder (-)-8-Phenylmenthyl hat,

mit Halogenmethylchinolinen der allgemeinen Formel VI 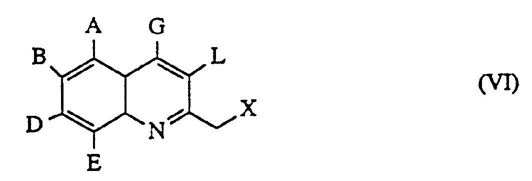 in welcher
A, B, D, E, G und L die oben angegebene Bedeutung haben

und
X für Halogen, vorzugsweise Chlor steht,

in inerten Lösemitteln, gegebenenfalls in Anwesenheit einer Base und/oder eines Hilfsstoffes, oder unter Phasentransferkatalyse verethert.

Die Herstellung kann durch das folgende Formelschema beispielhaft erläutert werden: 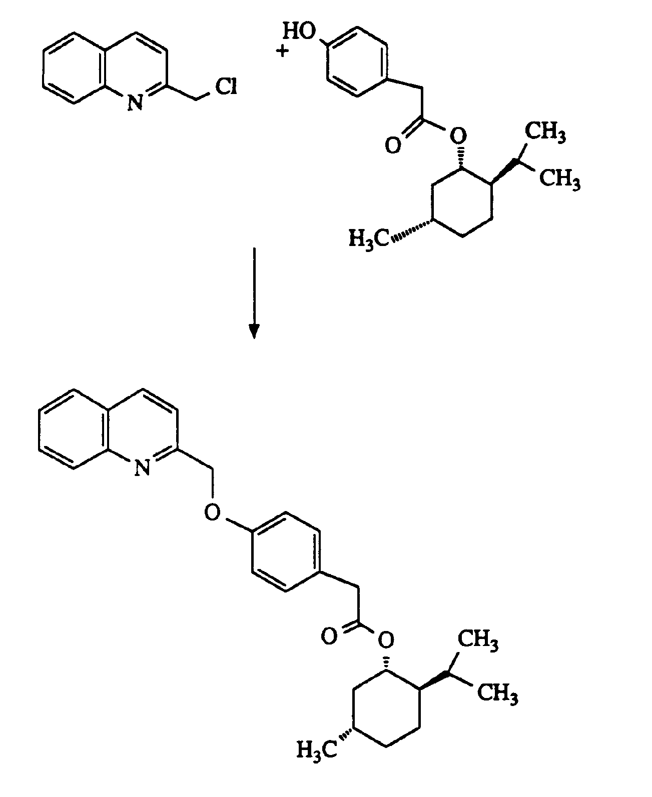 Die Veretherung kann in inerten organischen Lösemitteln gegebenenfalls in Anwesenheit einer Base durchgeführt werden. Lösemittel für die Veretherung können inerte organische Lösemittel sein, die sich unter den Reaktionsbedingungen nicht verändern. Hierzu gehören vorzugsweise Alkohole wie Ethanol, Propanol oder Isopropanol, Ether wie beispielsweise Dioxan, Tetrahydrofuran oder Diethylether, Halogenkohlenwasserstoffe wie Dichlormethan, Trichlormethan, Tetrachlormethan, 1,2-Dichlorethan oder Trichlorethylen, Kohlenwasserstoffe wie Benzol, Xylol, Toluol, Hexan, Cyclohexan oder Erdölfraktionen, Nitromethan, Dimethylformamid, Acetonitril, Aceton oder Hexamethylphosphorsäuretriamid. Ebenso ist es möglich, Gemische der Lösemittel einzusetzen. Bevorzugt sind Methylenchlorid und Isopropanol.

Als Basen für die Veretherung können anorganische oder organische Basen eingesetzt werden. Hierzu gehören vorzugsweise Alkalicarbonate wie Natriumcarbonat oder Kaliumcarbonat, Erdalkalicarbonate wie Calciumcarbonat, oder organische Amine (Trialkyl(C₁-C₆)amine) wie Triethylamin, oder Heterocyclen wie Pyridin, Methylpiperidin, Piperidin oder Morpholin. Ebenso können als Basen Hydride, wie Natriumhydrid, eingesetzt werden. Zur Aktivierung des eingesetzten Halogenids (111) ist es auch möglich, Alkalijodide, vorzugsweise Kaliumjodid zur Reaktionslösung zuzugeben.

Die Veretherung erfolgt im allgemeinen in einem Temperaturbereich von 0°C bis +150°C, bevorzugt von +10°C bis+100°C.

Die Veretherung wird im allgemeinen bei Normaldruck durchgeführt. Es ist aber auch möglich, das Verfahren bei Unterdruck oder Überdruck durchzuführen (z.B. in einem Bereich von 0,5 bis 5 bar).

Im allgemeinen setzt man 0,5 bis 5, bevorzugt 1 bis 2 Mol Halogenid (III), bezogen auf 1 Mol des Reaktionspartners ein. Die Base wird im allgemeinen in einer Menge von 0,5 bis 5 Mol, bevorzugt von 1 bis 3 Mol bezogen auf das Halogenid eingesetzt.

Die Phasentransferkatalyse wird im allgemeinen in einem der oben angegebenen Lösemittel, vorzugsweise in Dimethylformamid, Methylenchlorid oder Toluol, mit Kronenethern oder quarternären Ammoniumsalzen, vorzugsweise mit Tetrabutylammoniumjodid durchgeführt.

Die Verbindungen der allgemeinen Formel (VI) sind an sich bekannt oder können nach üblicher Methode hergestellt werden [vgl. Chem. Ber. 120, 649 (1987)].

Die Verbindungen der allgemeinen Formel (III) sind bekannt [vgl. Beilstein 5,19/5,24/5,29] oder können nach üblichen Methoden aus den entsprechenden Alkoholen oder Cycloalkenen hergestellt werden.

Die Verbindungen der allgemeinen Formel (VI) sind mit der freien OH-Funktion größtenteils neu und können beispielsweise aus den bekannten geschützten Derivaten (O-CH(CH₃)₂) durch Abspaltung der Schutzgruppe nach üblichen Methoden hergestellt werden (vgl. THL, Vol. 30, Nr. 3, S. 327 - 330 und J. Chem. Soc., Chem. Commun, 1986, S. 741 -742).

Im Rahmen des erfindungsgemäßen Verfahren werden die Verbindungen der allgemeinen Formel (V) mit dem entsprechenden chiralen Alkohol in Toluol und in Anwesenheit von p-Toluolsulfonsäure umgesetzt, wobei durch einen Überschuß des Alkohols Nebenreaktionen fast vollständig unterdrückt werden können.

Der chirale Alkohol wird in einer Menge von 0,5 mol bis 10 mol, bevorzugt von 1 mol bis 3 mol, bezogen auf die freie p-Hydroxyphenylessigsäure eingesetzt.

Der chirale Alkohol, insbesondere (+)- und (-)-Menthyl ist preiswert und in großen Mengen käuflich erhältlich.

Die folgenden Beispiele dienen der Verdeutlichung der Erfindung.

### Herstellungsbeispiele

### Beispiel 1

### 4-Hydroxyphenylessigsäure-(+)menthylester

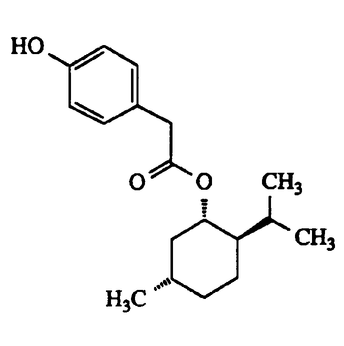

2,9 kg p-Hydroxyphenylessigsäure und 1,95 kg (+)-Menthol werden 16 Stunden mit 40 g p-Toluolsulfonsäure in 25 I Toluol zum Sieden erhitzt; dabei werden ca. 300 ml Wasser abgeschieden. Die Toluollösung wird mit 10 I gesättigter NaHC0₃-Lösung und 10 1 Wasser gewaschen und im Vakuum zur Trockene eingeengt. Man erhält die Titelverbindung als 5,4 kg öligen Rückstand (98 - 100% der Theorie) mit einem Gehalt von 91,8% (HPLC-Flächenprozente). Zu analytischen Zwecken wurde der Hydroxyphenylessigsäure-(+)-menthylester zweimal aus n-Hexan umkristallisiert. Schmp.: 50-51 °C
Drehwert: a_{D}²0 = 63,3 (c=1, CHCI₃)

### Beispiel 2

### 4-(Chinolin-2-yl-methoxy)phenylessigsäure-(+)-menthylester

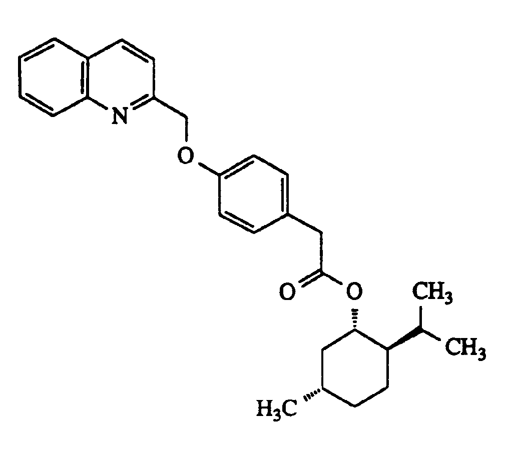

5,4 kg der Verbindung aus Beispiel 1, 28,7 kg Chinaldinchlorid, 3,87 kg Kaliumcarbonat und 400 g Kaliumjodid werden 20 Stunden in 181 siedendem Isopropanol umgesetzt. Durch Zugabe von 191 Wasser wird die Titelverbindung ausgefällt. Das Kristallisat wird geschleudert und mit 10 I Isopropanol/Wasser 1/1 und 8 I Wasser gewaschen.
Ausbeute: 5,44 kg (78% der Theorie).
Gehalt nach HPLC: 99,5%
Schmp.: 86°C
Drehwert: α_{D}20 = 45,0 (c = 1, CHCI₃)

### Beispiel 3

### (2R)-2-[4-(Chinolin-2-yl-methoxy)phenyl]-2-cyclopentylessigsäure-(+)-menthylester

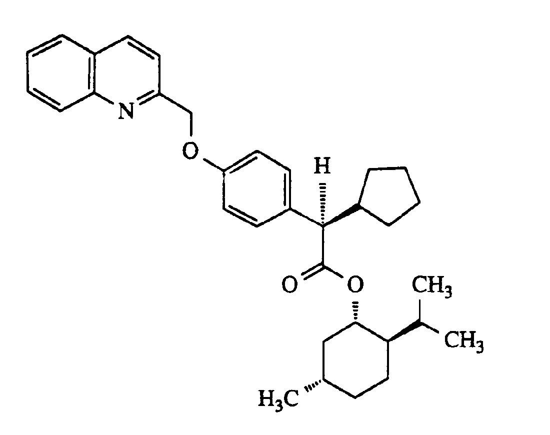

Zu einer Lösung von 5,44 kg der Verbindung aus Beispiel 2 und 2,06 kg Bromcyclopentan in 8,5 I Dimethylformamid wird unter Schutzgasatmosphäre zwischen -5°C und +5°C eine Lösung von 1,87 kg Kalium-tert.butylat in 5 I DMF zugegeben. Es wird noch 4 Stunden bei -5°C nachgerührt und dann mit 18,5 I Wasser versetzt. Das Rohprodukt wird abgeschleudert, 24 Stunden bei 60°C im Vakuum getrocknet und zweimal aus 7,5 I Ligroin umkristallisiert; bei der ersten Umkristallisation wird die heiße Ligroinlösung filtriert.
Ausbeute: 4,8 kg (76% der Theorie)
Gehalt nach HPLC: 99,7%
Diastereomerenüberschuß: 99,5%
Schmp.: 124°C
Drehwert: α_{D}20 = 26,6 (c=1, CHCI₃)

### Beispiel 4

### (2R)-2-[4(Chinolin-2-yl-methoxy)phenyl]-2-cyclopentylessigsäure

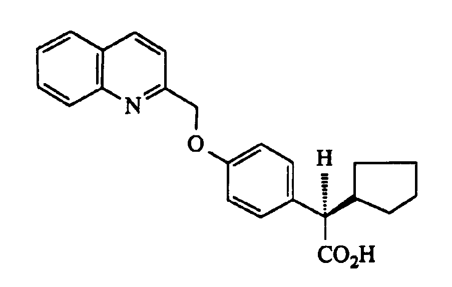

2,4 kg der Verbindung aus Beispiel 3 werden in einem siedenden Gemisch von 1 I konzentrierter Schwefelsäure und 5,6 I Eisessig während 4 Stunden hydrolysiert. Der Ansatz wird abgekühlt, mit 2 I Toluol versetzt und langsam in 15 1 Wasser und 6 I Toluol zulaufen lassen, wobei gleichzeitig durch Zugabe von ca. 1,7 I 45%iger Natronlauge neutralisiert wird. Die dabei ausfallende Titelverbindung wird abgeschleudert und aus 8 I Isopropanol umkristallisiert.
Ausbeute: 13,1 kg (75% der Theorie)
Gehalt nach HPLC: 99,9%
Enantiomerenüberschuß: 99,4%
Schmp.: 170-171 °C
Drehwert: α_{D}20 = 41,2 (c=1, CHCI₃)

### Beispiel 5

### Hydroxyphenylessigsäure-(-)-menthylester

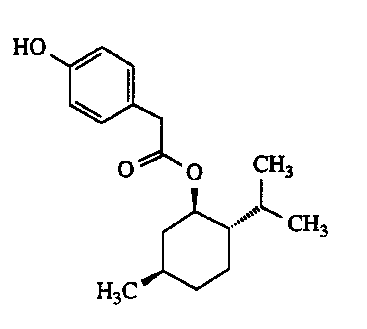

2,1 kg Hydroxyphenylessigsäure werden in 15 I siedendem Toluol unter Zusatz von 40 g Toluolsulfonsäure mit 2,5 kg (-)-Menthol verestert; das Reaktionswasser wird im Wasserabscheider ausgekreist. Wird kein Wasser mehr abgeschieden, kühlt man ab, wäscht mit 4 I gesättigter NaHC0₃ und 2 mal mit 4 I Wasser und engt im Vakuum zur Trockne ein. Man erhält die Titelverbindung als öligen Rückstand.
Ausbeute: 40,2 kg (100% der Theorie)
Gehalt nach HPLC: 95%
Schmp.: 50-51 °C
Drehwert: α_{D}20 = -62,5 (c=1, CHCI₃)

### Beispiel 6

### 4-(Chinolin-2-yl-methoxy)phenylessigsäure-(-)-menthylester

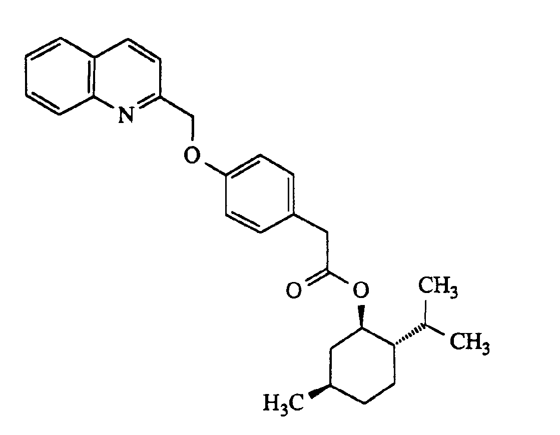

4,14 kg der Verbindung aus Beispiel 5 werden mit 2,48 kg Chinaldinchlorid, 2,94 kg Kaliumcarbonat, 150 g Kaliumjodid und 150 g Tetrabutylammoniumjodid in 15 I DMF 4 Stunden bei 50°C gerührt. Die Salze werden dann abfiltriert und mit 6 I Isopropanol nachgewaschen. Das Filtrat wird mit 2,5 I Wasser versetzt, dabei kristallisiert die Titelverbindung aus. Sie wird abgeschleudert, mit 8 I Isopropanol/Wasser 1:1, mit 6 I Wasser nachgewaschen und im Vakuum bei 50°C getrocknet.
Ausbeute: 5,42 kg (88% der Theorie)
Gehalt nach HPLC: 99%
Schmp.: 86°C
Drehwert: α_{D}²⁰ = -44,0 (c=1, CHCI₃)

### Beispiel 7

### (2R)-2-[4-(Chinolin-2-yl-methoxy)phenyl]-2-cycloheptylessigsäure-(-)-menthylester

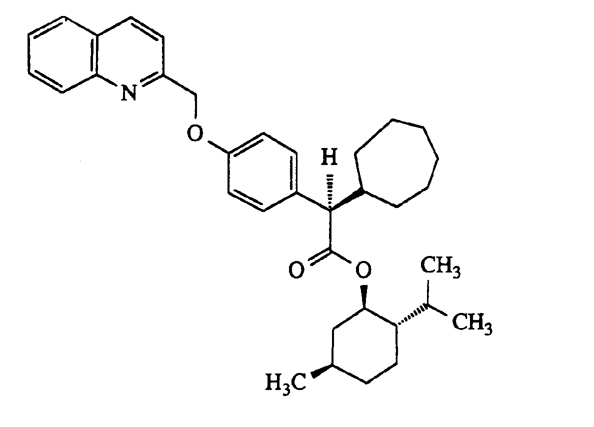

1,3 kg der Verbindung aus Beispiel 6 werden mit 580 g Bromcycloheptan in 2,4 I DMF unter Feuchtigkeitsausschluß bei -5°C bis +5°C gerührt. Während ca. 2 Stunden wird eine Lösung von 400 g Kalium-tert.butylat in 1,61 DMF zugegeben. Es wird noch vier Stunden zwischen -20°C und -10°C nachgerührt und dann mit 7,5 I Wasser versetzt, dabei fällt die Titelverbindung als Rohprodukt aus. Das Rohprodukt wird abgeschleudert, bei 50°C im Vakuum getrocknet und zweimal mit 6 I Ligroin umkristallisiert; bei der ersten Umkristallisation wird heiß filtriert.
Ausbeute: 1,02 kg (64% der Theorie)
Gehalt nach HPLC: >99%
Diastereomerenüberschuß: >99%
Schmp.: 127°C
Drehwert: α_{D}20 = -44,3 (c=1, CH₂Cl₂)

### Beispiel 8

### (2R)-2-[4-(Chinolin-2-yl-methoxy)phenyl]-2-cycloheptylessigsäure

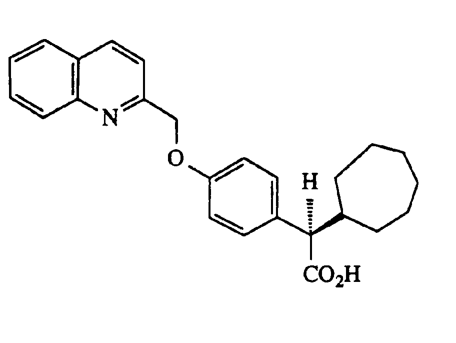

1 kg der Verbindung aus Beispiel 7 wird 3 bis 4 Stunden in einem siedenden Gemisch von 9 I 98%iger Ameisensäure und 0,8 I 48%iger Bromwasserstoffsäure gerührt. Eine sich im Destillat abscheidende leichte Phase wird dabei abgetrennt. Die Reaktionslösung wird abgekühlt und zu einer Mischung von 750 ml 45%iger Natronlauge, 7,5 I Wasser und 2 I Isopropanol zugegeben. Das dabei ausfallende Rohprodukt wird abgeschleudert und aus einer Mischung von 3,6 I Isopropanol und 1,2 I Wasser umkristallisiert.
Ausbeute: 624 g (85% der Theorie)
Gehalt nach HPLC: >99%
Enantiomerenüberschuß: >99,5%
Schmp.: 170-172°C
Drehwert: α_{D}20 = -27,5 (c=1, CH₂Cl₂)

## Patentansprüche (Patentansprüche für folgende(n) Vertragsstaat(en) : AT, BE, CH, DE, DK, FR, GB, GR, IT, LI, LU, NL, SE)

1. Verfahren zur Herstellung von enantiomerenreinen (Chinolin-2-yl-methoxy)phenylessigsäuren der allgemeinen Formel 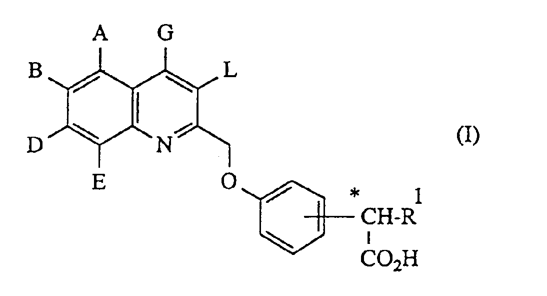 in welcher
A, B, D, E, G und L gleich oder verschieden sind und für Wasserstoff, Hydroxy, Halogen, Carboxy, Nitro, Trifluormethyl, Trifluormethoxy oder
für geradkettiges oder verzweigtes Alkyl oder Alkoxy mit jeweils bis zu 8 Kohlenstoffatomen stehen, oder
für Aryl mit 6 bis 10 Kohlenstoffatomen stehen, das gegebenenfalls durch Halogen, Hydroxy, Nitro oder Cyano substituiert ist,
und
R¹ für Cycloalkyl mit 4 bis 12 Kohlenstoffatomen steht,
dadurch gekennzeichnet, daß man Verbindungen der allgemeinen Formel 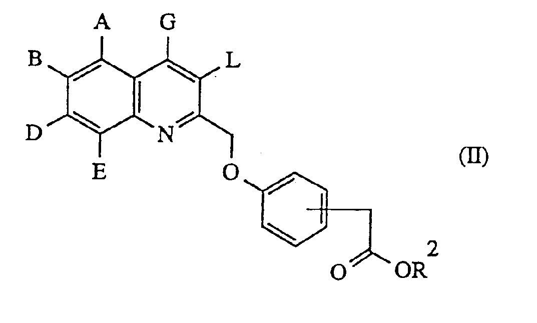 in welcher
A, B, D, E, G und L die oben angegebene Bedeutung haben,
und
R² die Bedeutung (+)- oder (-)-Menthyl, (+)- oder (-)-Bornyl, (+)- oder (-)-Isobornyl oder (-)-8-Phenylmenthyl hat,
zunächst mit Verbindungen der allgemeinen Formel (III) 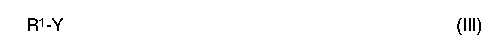 in welcher
R¹ die oben angegebene Bedeutung hat
und
Y für ein Brom, Chlor,
Jod, Mesyl, Tosyl oder Trifluormethylsulfonyl steht,
in inerten Lösemitteln in Anwesenheit einer Base durch diastereoselektive Alkylierung in die Verbindungen der allgemeinen Formel (IV) 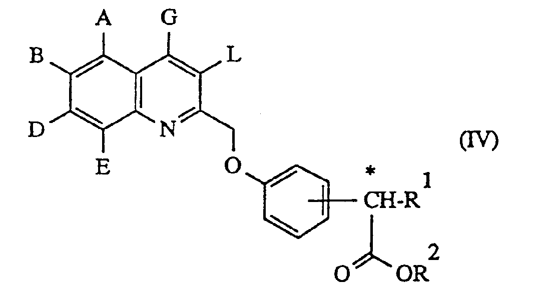 in welcher
A, B, D, E, G, R¹ und R² die angegebene Bedeutung haben,
überführt
und in einem zweiten Schritt den Rest R² gezielt mit Säuren ohne Racemisierung abspaltet.

2. Verfahren nach Anspruch 1 zur Herstellung von enantiomerenreinen (Chinolin-2-yl-methoxy)phenylessigsäuren der Formel (I) worin
A, B, D, E, G und L gleich oder verschieden sind und
für Wasserstoff, Fluor, Chlor, Brom, Trifluormethyl oder für geradkettiges oder verzweigtes Alkyl mit bis zu 6 Kohlenstoffatomen stehen,
und
R¹ für Cyclopentyl, Cyclophenyl, Cycloheptyl oder Cyclooctyl steht.

3. Verfahren nach Anspruch 1 zur Herstellung von enantiomerenreinen (Chinolin-2-yl-methoxy)phenylessigsäuren der Formel I in welcher
A, B, D, E, G und L für Wasserstoff stehen und
R¹ für Cyclopentyl, Cyclohexyl oder Cycloheptyl steht.

4. Verfahren nach den Ansprüchen 1 bis 3 dadurch gekennzeichnet, daß Y in Formel 111 für Jod oder Brom steht.

5. Verfahren nach den Ansprüchen 1 bis 4 dadurch gekennzeichnet, daß R² die Bedeutung (+)- oder (-)-Menthyl hat.

6. Verfahren nach den Ansprüchen 1 bis 5 dadurch gekennzeichnet, daß man als Base bei der Alkylierung Alkalihydride, Alkaliamide, Alkalialkoholate, organische Amine oder lithiumorganische Verbindungen einsetzt.

7. Verfahren nach den Ansprüchen 1 bis 6 dadurch gekennzeichnet, daß man als Säuren zur Abspaltung des chiralen Restes R² Essigsäure, Ameisensäure, Bromwasserstoffsäure und/oder Schwefelsäure verwendet.

8. Verbindungen der allgemeinen Formel 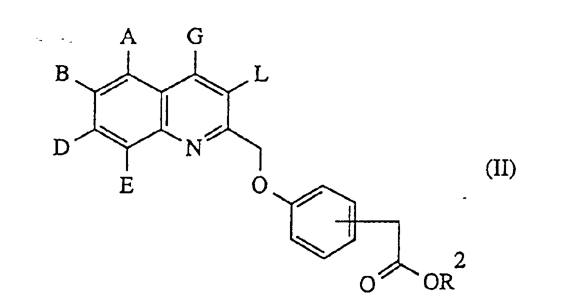 in welcher
A, B, D, E, G, L gleich oder verschieden sind und
für Wasserstoff, Hydroxy, Halogen, Carboxy, Nitro, Trifluormethyl, Trifluormethoxy oder
für geradkettiges oder verzweigtes Alkyl oder Alkoxy mit jeweils bis zu 8 Kohlenstoffatomen stehen, oder
für Aryl mit 6 bis 10 Kohlenstoffatomen stehen das gegebenenfalls durch Halogen, Hydroxy, Nitro oder Cyano substituiert ist,
und
R² die Bedeutung (+)- oder (-)-Menthyl, (+)- oder (-)-Bornyl, (+)- oder (-)-Isobornyl oder (-)-8-Phenylmenthyl hat.

9. Verbindungen nach Anspruch 8
worin
R2 für (+)- oder (-)-Menthyl steht.

10. Verfahren zur Herstellung von Verbindungen nach Anspruch 8 dadurch gekennzeichnet, daß man Hydroxyphenylessigsäurederivate der Formel 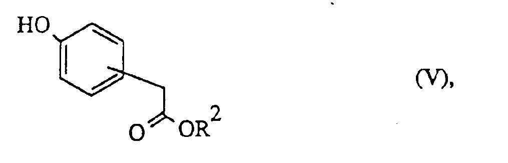 in welcher
R² die Bedeutung (+)- oder (-)-Menthyl, (+)- oder (-)-Bornyl, (+)- oder (-)-Isobornyl oder (-)-8-Phenylmenthyl hat
mit Halogenmethylchinolinen der allgemeinen Formel VI 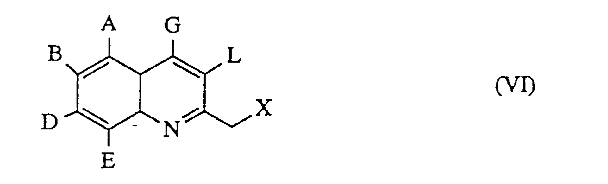 in welcher
A, B, D, E, G und L die oben angegebene Bedeutung haben
und
X für Halogen steht,
in inerten Lösemitteln, gegebenenfalls in Anwesenheit einer Base und/oder eines Hilfsstoffes, oder unter Phasentransferkatalyse verethert.

## Patentansprüche (Patentansprüche für folgende(n) Vertragsstaat(en) : ES)

1. Verfahren zur Herstellung von enantiomerenreinen (Chinolin-2-yl-methoxy)phenylessigsäuren der allgemeinen Formel 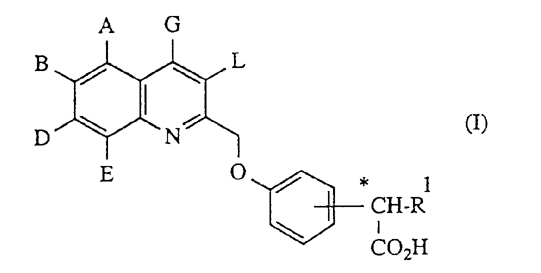 in welcher
A, B, D, E, G und L gleich oder verschieden sind und
für Wasserstoff, Hydroxy, Halogen, Carboxy, Nitro, Trifluormethyl, Trifluormethoxy oder für geradkettiges oder verzweigtes Alkyl oder Alkoxy mit jeweils bis zu 8 Kohlenstoffatomen stehen, oder
für Aryl mit 6 bis 10 Kohlenstoffatomen stehen, das gegebenenfalls durch Halogen, Hydroxy, Nitro oder Cyano substituiert ist,
und
R¹ für Cycloalkyl mit 4 bis 12 Kohlenstoffatomen steht,
dadurch gekennzeichnet, daß man Verbindungen der allgemeinen Formel 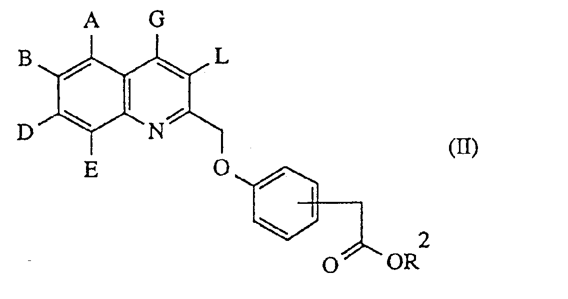 in welcher
A, B, D, E, G und L die oben angegebene Bedeutung haben,
und
R² die Bedeutung (+)- oder (-)-Menthyl, (+)- oder (-)-Bornyl, (+)- oder (-)-Isobornyl oder (-)-8-Phenylmenthyl hat,
zunächst mit Verbindungen der allgemeinen Formel (III) 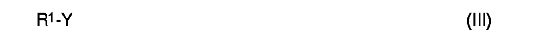 in welcher
R¹ die oben angegebene Bedeutung hat
und
Y für Brom, Chlor, Jod, Mesyl, Tosyl oder Trifluormethylsulfonyl steht,
in inerten Lösemitteln in Anwesenheit einer Base durch diastereoselektive Alkylierung in die Verbindungen der allgemeinen Formel (IV) 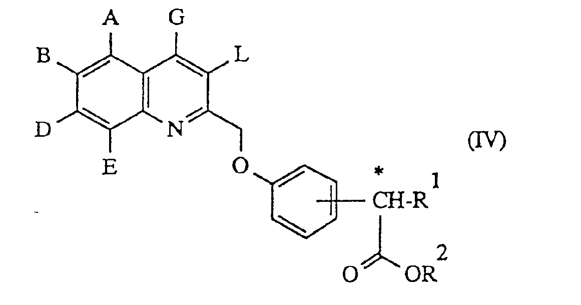 in welcher
A, B, D, E, G, R¹ und R² die angegebene Bedeutung haben,
überführt
und in einem zweiten Schritt den Rest R² gezielt mit Säuren ohne Racemisierung abspaltet.

2. Verfahren nach Anspruch 1 zur Herstellung von enantiomerenreinen (Chinolin-2-yl-methoxy)phenylessigsäuren der Formel (I)
worin
A, B, D, E, G und L gleich oder verschieden sind und
für Wasserstoff, Fluor, Chlor, Brom, Trifluormethyl oder für geradkettiges oder verzweigtes Alkyl mit bis zu 6 Kohlenstoffatomen stehen,
und
R¹ für Cyclopentyl, Cyclophenyl, Cycloheptyl oder Cyclooctyl steht.

3. Verfahren nach Anspruch 1 zur Herstellung von enantiomerenreinen (Chinolin-2-yl-methoxy)phenylessigsäuren der Formel I in welcher
A, B, D, E, G und L für Wasserstoff stehen und
R¹ für Cyclopentyl, Cyclohexyl oder Cycloheptyl steht.

4. Verfahren nach den Ansprüchen 1 bis 3 dadurch gekennzeichnet, daß Y in Formel 111 für Jod oder Brom steht.

5. Verfahren nach den Ansprüchen 1 bis 4 dadurch gekennzeichnet, daß R² die Bedeutung (+)- oder (-)-Menthyl hat.

6. Verfahren nach den Ansprüchen 1 bis 5 dadurch gekennzeichnet, daß man als Base bei der Alkylierung Alkalihydride, Alkaliamide, Alkalialkoholate, organische Amine oder lithiumorganische Verbindungen einsetzt.

7. Verfahren nach den Ansprüchen 1 bis 6 dadurch gekennzeichnet, daß man als Säuren zur Abspaltung des chiralen Restes R² Essigsäure, Ameisensäure, Bromwasserstoffsäure und/oder Schwefelsäure verwendet.

8. Verfahren zur Herstellung von Verbindungen der allgemeinen Formel II 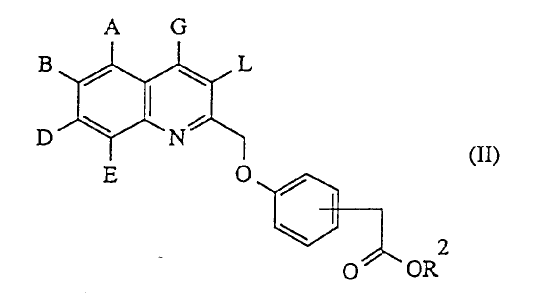 in welcher
A, B, D, E, G, L gleich oder verschieden sind und
für Wasserstoff, Hydroxy, Halogen, Carboxy, Nitro, Trifluormethyl, Trifluormethoxy oder
für geradkettiges oder verzweigtes Alkyl oder Alkoxy mit jeweils bis zu 8 Kohlenstoffatomen stehen, oder
für Aryl mit 6 bis 10 Kohlenstoffatomen stehen, das gegebenenfalls durch Halogen, Hydroxy, Nitro oder Cyano substituiert ist,
und
R² die Bedeutung (+)- oder (-)-Menthyl, (+)- oder (-)-Bornyl, (+)- oder (-)-Isobornyl oder (-)-8-Phenylmenthyl hat dadurch gekennzeichnet, daß man Hydroxyphenylessigsäurederivate der Formel 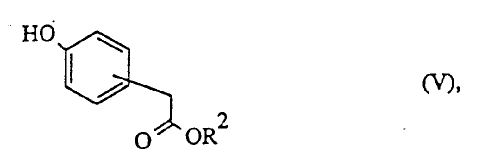 in welcher
R² die Bedeutung (+)- oder (-)-Methyl, (+)- oder (-)-Bornyl, (+)- oder (-)-Isobornyl oder (-)-8-Phenylmethyl hat
mit Halogenmethylchinolinen der allgemeinen Formel VI 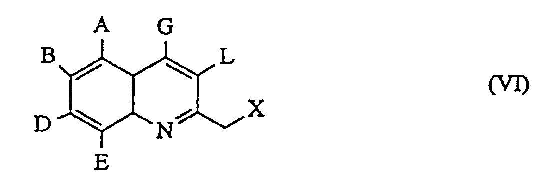 in welcher
A, B, D, E, G und L die oben angegebene Bedeutung haben
und
X für Halogen steht,
in inerten Lösemitteln, gegebenenfalls in Anwesenheit einer Base und/oder eines Hilfsstoffes, oder unter Phasentransferkatalyse verethert.

## Claims (Claims for the following Contracting State(s) : AT, BE, CH, DE, DK, FR, GB, GR, IT, LI, LU, NL, SE)

1. Process for the preparation of enantiomerically pure (quinolin-2-yl-methoxy)phenylacetic acids of the general formula 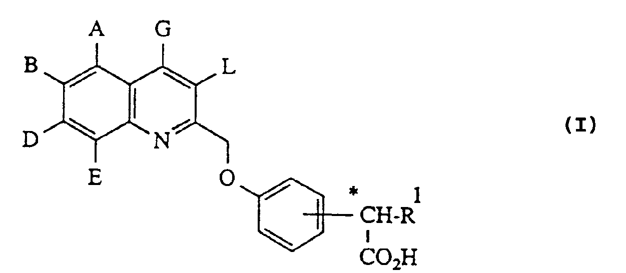 in which
A, B, D, E, G and L are identical or different and represent hydrogen, hydroxyl, halogen, carboxyl, nitro, trifluoromethyl, trifluoromethoxy or represent straight-chain or branched alkyl or alkoxy each having up to 8 carbon atoms, or represent aryl having 6 to 10 carbon atoms, which is optionally substituted by halogen, hydroxyl, nitro or cyano,
and
R¹ represents cycloalkyl having 4 to 12 carbon atoms,
characterised in that
compounds of the general formula 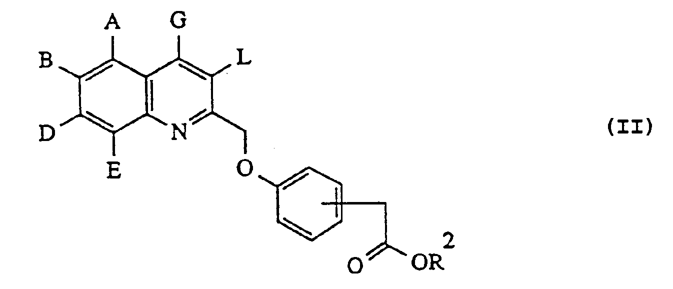 in which
A, B, D, E, G and L have the abovementioned meaning,
and
R² has the meaning (+)- or (-)-menthyl, (+)- or (-)-bornyl, (+)- or (-)-isobornyl or (-)-8-phenylmenthyl,
are first converted with compounds of the general formula (III) 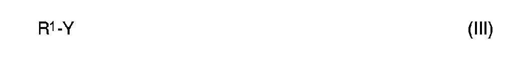 in which
R¹ has the abovementioned meaning
and
Y represents bromine, chlorine, iodine, mesyl, tosyl or trifluoromethylsulphonyl,
by diastereoselective alkylation in inert solvents in the presence of a base into compounds of the general formula (IV) 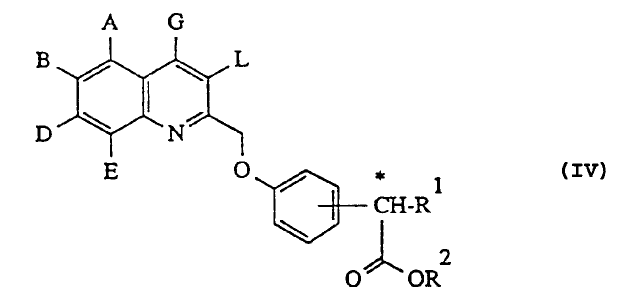 in which
A, B, D, E, G, R¹ and R² have the abovementioned meaning,
and in a second step the radical R² is specifically removed with acids without racemisation.

2. Process according to Claim 1 for the preparation of enantiomerically pure (quinolin-2-yl-methoxy)phenylacetic acids of the formula (I)
in which
A, B, D, E, G and L are identical or different and
represent hydrogen, fluorine, chlorine, bromine, trifluoromethyl or
represent straight-chain or branched alkyl having up to 6 carbon atoms
and
R¹ represents cyclopentyl, cyclohexyl, cycloheptyl or cyclooctyl.

3. Process according to Claim 1 for the preparation of enantiomerically pure (quinolin-2-yl-methoxy)phenylacetic acids of the formula I in which
A, B, D, E, G and L represent hydrogen and
R¹ represents cyclopentyl, cyclohexyl or cycloheptyl.

4. Process according to Claims 1 to 3, characterised in that Y in formula III represents iodine or bromine.

5. Process according to Claims 1 to 4, characterised in that
R² has the meaning (+)- or (-)-menthyl.

6. Process according to Claims 1 to 5, characterised in that in the alkylation alkali metal hydrides, alkali metal amides, alkali metal alkoxides, organic amines or organolithium compounds are employed as base.

7. Process according to Claims 1 to 6, characterised in that acetic acid, formic acid, hydrobromic acid and/or sulphuric acid are used as acids for the removal of the chiral radical R^{2.}

8. Compounds of the general formula 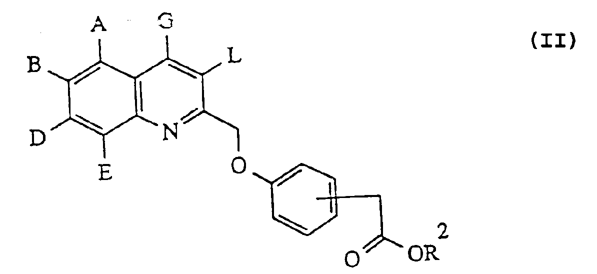 in which
A, B, D, E, G and L are identical or different and represent hydrogen, hydroxyl, halogen, carboxyl, nitro, trifluoromethyl, trifluoromethoxy or
represent straight-chain or branched alkyl or alkoxy each having up to 8 carbon atoms, or represent aryl having 6 to 10 carbon atoms, which is optionally substituted by halogen, hydroxyl, nitro or cyano, and
R² has the meaning (+)- or (-)-methyl, (+)- or (-)-bornyl, (+)- or (-)-isobornyl or (-)-8-phenylmenthyl,

9. Compounds according to Claim 8,
in which
R2 represents (+)- or (-)-menthyl.

10. Process for the preparation of compounds according to Claim 8, characterised in that hydroxyphenylacetic acid derivatives of the formula 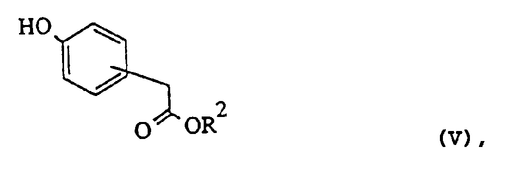 in which
R² has the meaning (+)- or (-)-methyl, (+)- or (-)-bornyl, (+)- or (-)-isobornyl or (-)-8-phenylmenthyl,
are etherified with halogenomethylquinolines of the general formula VI 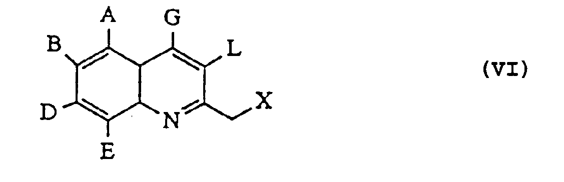 in which
A, B, D, E, G and L have the abovementioned meaning
and X represents halogen,
in inert solvents, if appropriate in the presence of a base and/or of an auxiliary, or under phase transfer catalysis.

## Claims (Claims for the following Contracting State(s) : ES)

1. Process for the preparation of enantiomerically pure (quinolin-2-yl-methoxy)phenylacetic acids of the general formula 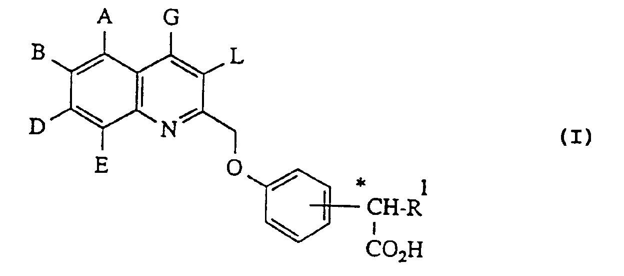 in which
A, B, D, E, G and L are identical or different and represent hydrogen, hydroxyl, halogen, carboxyl, nitro, trifluoromethyl, trifluoromethoxy or represent straight-chain or branched alkyl or alkoxy each having up to 8 carbon atoms, or represent aryl having 6 to 10 carbon atoms, which is optionally substituted by halogen, hydroxyl, nitro or cyano,
and
R¹ represents cycloalkyl having 4 to 12 carbon atoms,
characterised in that
compounds of the general formula 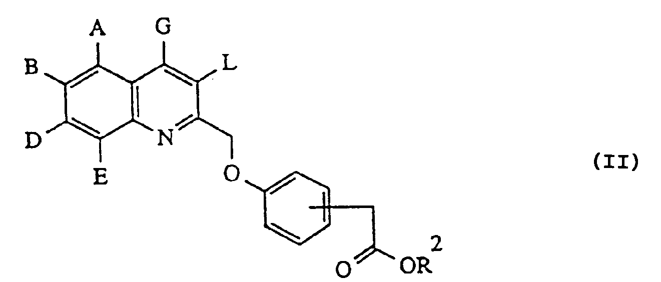 in which
A, B, D, E, G and L have the abovementioned meaning,
and
R² has the meaning (+)- or (-)-menthyl, (+)- or (-)-bornyl, (+)- or (-)-isobornyl or (-)-8-phenylmenthyl,
are first converted with compounds of the general formula (III) 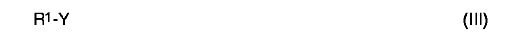 in which
R¹ has the abovementioned meaning
and
Y represents bromine, chlorine, iodine, mesyl, tosyl or trifluoromethylsulphonyl,
by diastereoselective alkylation in inert solvents in the presence of a base into compounds of the general formula (IV) 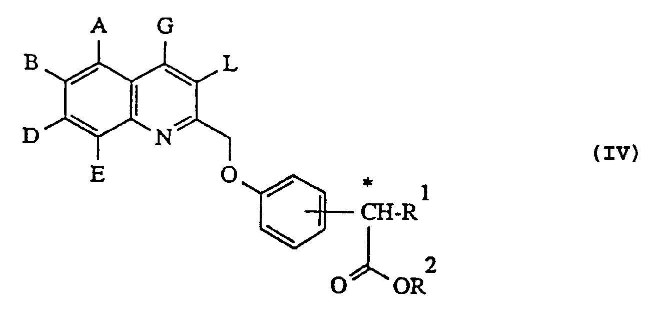 in which
A, B, D, E, G, R¹ and R² have the abovementioned meaning,
and in a second step the radical R² is specifically removed with acids without racemisation.

2. Process according to Claim 1 for the preparation of enantiomerically pure (quinolin-2-yl-methoxy)phenylacetic acids of the formula (I)
in which
A, B, D, E, G and L are identical or different and
represent hydrogen, fluorine, chlorine, bromine, trifluoromethyl or
represent straight-chain or branched alkyl having up to 6 carbon atoms
and
R¹ represents cyclopentyl, cyclohexyl, cycloheptyl or cyclooctyl.

3. Process according to Claim 1 for the preparation of enantiomerically pure (quinolin-2-yl-methoxy)phenylacetic acids of the formula I in which
A, B, D, E, G and L represent hydrogen and
R¹ represents cyclopentyl, cyclohexyl or cycloheptyl.

4. Process according to Claims 1 to 3, characterised in that Y in formula III represents iodine or bromine.

5. Process according to Claims 1 to 4, characterised in that
R² has the meaning (+)- or (-)-menthyl.

6. Process according to Claims 1 to 5, characterised in that in the alkylation alkali metal hydrides, alkali metal amides, alkali metal alkoxides, organic amines or organolithium compounds are employed as base.

7. Process according to Claims 1 to 6, characterised in that acetic acid, formic acid, hydrobromic acid and/or sulphuric acid are used as acids for the removal of the chiral radical R^{2.}

8. Process for the preparation of compounds of the general formula I 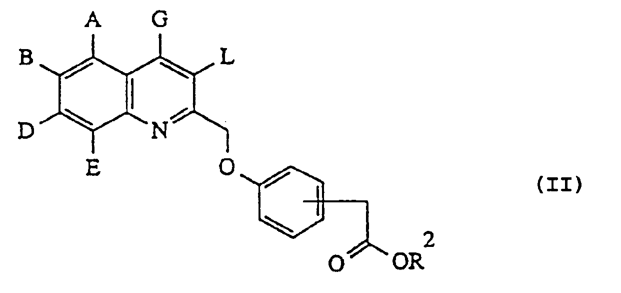 in which
A, B, D, E, G and L are identical or different and represent hydrogen, hydroxyl, halogen, carboxyl, nitro, trifluoromethyl, trifluoromethoxy or
represent straight-chain or branched alkyl or alkoxy each having up to 8 carbon atoms, or represent aryl having 6 to 10 carbon atoms, which is optionally substituted by halogen, hydroxyl, nitro or cyano,
and
R² has the meaning (+)- or (-)-menthyl, (+)- or (-)-bornyl, (+)- or (-)-isobornyl or (-)-8-phenylmenthyl, characterised in that hydroxyphenylacetic acid derivatives of the formula 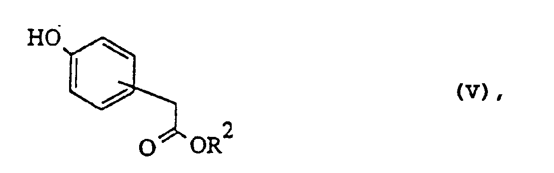 in which
R² has the meaning (+)- or (-)-menthyl, (+)- or (-)-bornyl, (+)- or (-)-isobornyl or (-)-8-phenylmenthyl,
are etherified with halogenomethylquinolines of the general formula VI 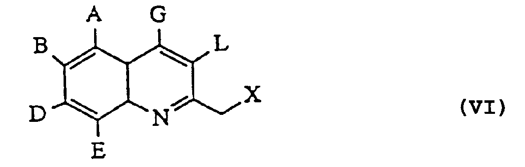 in which
A, B, D, E, G and L have the abovementioned meaning
and
X represents halogen,
in inert solvents, if appropriate in the presence of a base and/or of an auxiliary, or under phase transfer catalysis.

## Revendications (Revendications pour l'(les) Etat(s) contractant(s) suivant(s) : AT, BE, CH, DE, DK, FR, GB, GR, IT, LI, LU, NL, SE)

1. Procédé de production d'acides (quinoléine-2-ylméthoxy)phénylacétiques de pureté énantiomérique, de formule générale 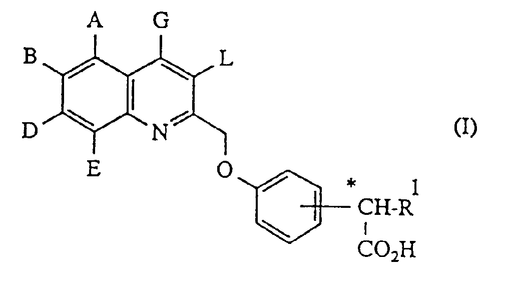 dans laquelle
A, B, D, E, G et L sont identiques ou différents et représentent
de l'hydrogène, un groupe hydroxy, un halogène, un groupe carboxy, nitro, trifluorométhyle, trifluorométhoxy, ou
un groupe alkyle ou un groupe alkoxy linéaire ou ramifié ayant chacun jusqu'à 8 atomes de carbone, ou
un groupe aryle ayant 6 à 10 atomes de carbone, qui est substitué le cas échéant par un halogène, un radical hydroxy, nitro ou cyano,
et
R¹ est un groupe cycloalkyle ayant 4 à 12 atomes de carbone,
caractérisé en ce qu'on transforme des composés de formule générale 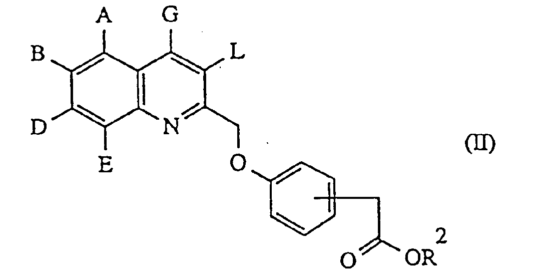 dans laquelle
A, B, D, E, G et L ont la définition indiquée ci-dessus, et
R² représente un groupe (+)- ou (-)-menthyle, (+)-ou (-)-bornyle, (+)- ou (-)-isobornyle ou (-)-8-phénylmenthyle,
tout d'abord avec des composés de formule générale (III) 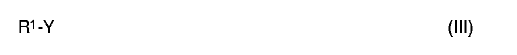
dans laquelle
R¹ a la définition indiquée ci-dessus et
et
Y représente le brome, le chlore, l'iode, un groupe mésyle, tosyle ou trifluorométhylsulfonyle,
dans des solvants inertes en présence d'une base par alkylation diastéréosélective, en les composés de formule générale (IV) 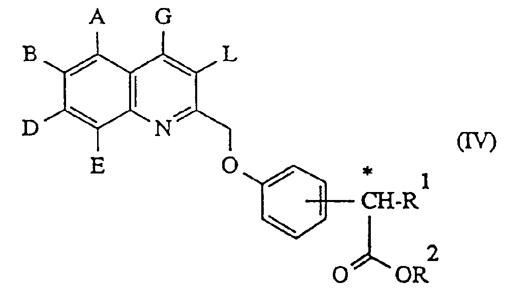 dans laquelle
A, B, D, E, G, R¹ et R² ont la définition indiquée ci-dessus, et on élimine dans une seconde étape le reste R² de façon appropriée avec des acides, sans racémisation.

2. Procédé suivant la revendication 1, pour l'obtention d'acides (quinoléine-2-ylméthoxy)phénylacétiques de formule (I) de pureté énantiomérique
dans laquelle
A, B, D, E, G et L sont identiques ou différents et représentent
de l'hydrogène, du fluor, du chlore, du brome, un groupe trifluorométhyle ou bien
un groupe alkyle linéaire ou ramifié ayant jusqu'à 6 atomes de carbone,
et
R¹ est un groupe cyclopentyle, cyclophényle, cycloheptyle ou cyclooctyle.

3. Procédé suivant la revendication 1, pour l'obtention d'acides (quinoléine-2-ylméthoxy)phénylacétiques de pureté énantiomérique de formule I dans laquelle
A, B, D, E, G et L représentent de l'hydrogène et
R¹ est un groupe cyclopentyle, cyclohexyle ou cycloheptyle.

4. Procédé suivant les revendications 1 à 3, caractérisé en ce que Y dans la formule I représente de l'iode ou du brome.

5. Procédé suivant les revendications 1 à 4, caractérisé en ce que R² désigne un groupe (+)-menthyle ou (-)-menthyle.

6. Procédé suivant les revendications 1 à 5, caractérisé en ce qu'on utilise comme base dans l'alkylation, des hydrures alcalins, des amidures alcalins, des alcoolates alcalins, des amines organiques ou des composés organiques de lithium.

7. Procédé suivant les revendications 1 à 6, caractérisé en ce qu'on utilise comme acides pour l'élimination du reste chiral R², l'acide acétique, l'acide formique, l'acide bromhydrique et/ou l'acide sulfurique.

8. Composés de formule générale 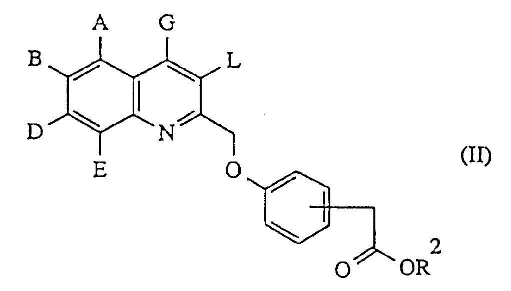 dans laquelle
A, B, D, E, G, L sont identiques ou différents et représentent
de l'hydrogène, un groupe hydroxy, un halogène, un groupe carboxy, nitro, trifluorométhyle, trifluorométhoxy, ou
un groupe alkyle ou un groupe alkoxy linéaire ou ramifié ayant chacun jusqu'à 8 atomes de carbone, ou
un groupe aryle de 6 à 10 atomes de carbone, qui est substitué le cas échéant par un halogène, un radical hydroxy, nitro ou cyano,
et
R² représente un groupe (+)- ou (-)-menthyle, (+)-ou (-)-bornyle, (+)- ou (-)-isobornyle ou (-)-8-phénylmenthyle.

9. Composés suivant la revendication 8, dans lesquels
R2 est un groupe (+)- ou (-)-menthyle.

10. Procédé de production de composés suivant la revendication 8, caractérisé en ce qu'on éthérifie des dérivés d'acide hydroxyphénylacétique de formule 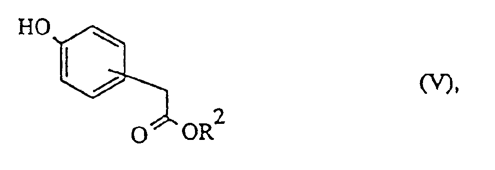 dans laquelle
R² est un groupe (+)- ou (-)-menthyle, (+)- ou (-)-bornyle, (+)- ou (-)-isobornyle ou (-)-8-phénylmenthyle
avec des halogénométhylquinoléines de formule générale VI 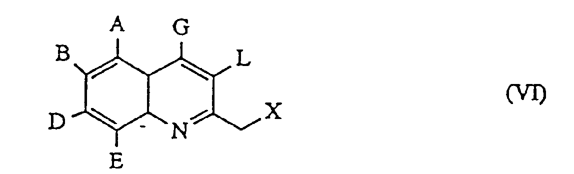 dans laquelle
A, B, D, E, G et L ont la définition indiquée ci-dessus
et
X représente un halogène,
dans des solvants inertes, le cas échéant en présence d'une base et/ou d'une substance auxiliaire, ou par catalyse avec transfert de phase.

## Revendications (Revendications pour l'(les) Etat(s) contractant(s) suivant(s) : ES)

1. Procédé de production d'acides (quinoléine-2-ylméthoxy)phénylacétiques de pureté énantiomérique, de formule générale 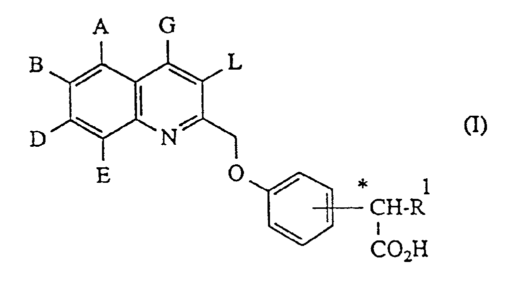 dans laquelle
A, B, D, E, G et L sont identiques ou différents et représentent
de l'hydrogène, un groupe hydroxy, un halogène, un groupe carboxy, nitro, trifluorométhyle, trifluorométhoxy, ou
un groupe alkyle ou un groupe alkoxy linéaire ou ramifié ayant chacun jusqu'à 8 atomes de carbone, ou
un groupe aryle ayant 6 à 10 atomes de carbone, qui est substitué le cas échéant par un halogène, un radical hydroxy, nitro ou cyano,
et
R¹ est un groupe cycloalkyle ayant 4 à 12 atomes de carbone,
caractérisé en ce qu'on transforme des composés de formule générale 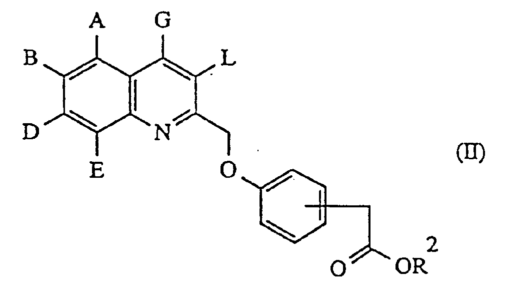 dans laquelle
A, B, D, E, G et L ont la définition indiquée ci-dessus,
et
R² représente un groupe (+)- ou (-)-menthyle, (+)-ou (-)-bornyle, (+)- ou (-)-isobornyle ou (-)-8-phénylmenthyle,
tout d'abord avec des composés de formule générale (III) 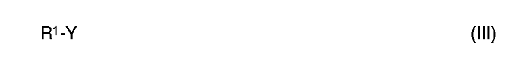 dans laquelle
R¹ a la définition indiquée ci-dessus et
et
Y représente le brome, le chlore, l'iode, un groupe mésyle, tosyle ou trifluorométhylsulfonyle,
dans des solvants inertes en présence d'une base par alkylation diastéréosélective, en les composés de formule générale (IV) 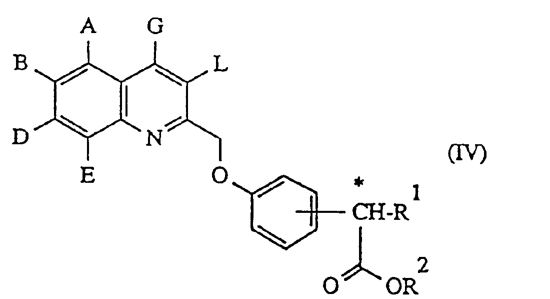 dans laquelle
A, B, D, E, G, R¹ et R² ont la définition indiquée ci-dessus, et on élimine dans une seconde étape le reste R² de façon appropriée avec des acides, sans racémisation.

2. Procédé suivant la revendication 1, pour l'obtention d'acides (quinoléine-2-ylméthoxy)phénylacétiques de formule (I) de pureté énantiomérique
dans laquelle
A, B, D, E, G et L sont identiques ou différents et représentent
de l'hydrogène, du fluor, du chlore, du brome, un groupe trifluorométhyle ou bien
un groupe alkyle linéaire ou ramifié ayant jusqu'à 6 atomes de carbone,
et
R¹ est un groupe cyclopentyle, cyclophényle, cycloheptyle ou cyclooctyle.

3. Procédé suivant la revendication 1, pour l'obtention d'acides (quinoléine-2-ylméthoxy)phénylacétiques de pureté énantiomérique de formule I dans laquelle
A, B, D, E, G et L représentent de l'hydrogène et
R¹ est un groupe cyclopentyle, cyclohexyle ou cycloheptyle.

4. Procédé suivant les revendications 1 à 3, caractérisé en ce que Y dans la formule I représente de l'iode ou du brome.

5. Procédé suivant les revendications 1 à 4, caractérisé en ce que R² désigne un groupe (+)-menthyle ou (-)-menthyle.

6. Procédé suivant les revendications 1 à 5, caractérisé en ce qu'on utilise comme base dans l'alkylation, des hydrures alcalins, des amidures alcalins, des alcoolates alcalins, des amines organiques ou des composés organiques de lithium.

7. Procédé suivant les revendications 1 à 6, caractérisé en ce qu'on utilise comme acides pour l'élimination du reste chiral R², l'acide acétique, l'acide formique, l'acide bromhydrique et/ou l'acide sulfurique.

8. Procédé de production de composés de formule générale Il 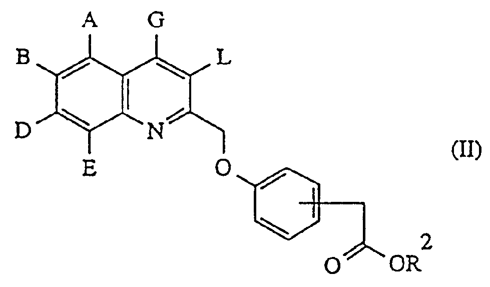 dans laquelle
A, B, D, E, G, L sont identiques ou différents et représentent
de l'hydrogène, un groupe hydroxy, un halogène, un groupe carboxy, nitro, trifluorométhyle, trifluorométhoxy, ou
un groupe alkyle ou un groupe alkoxy linéaire ou ramifié ayant chacun jusqu'à 8 atomes de carbone, ou
un groupe arylede 6 à 10 atomes de carbone, qui est substitué le cas échéant par un halogène, un radical hydroxy, nitro ou cyano,
et
R² représente un groupe (+)- ou (-)-menthyle, (+)-ou (-)-bornyle, (+)- ou (-)-isobornyle ou (-)-8-phénylmenthyle
caractérisé en ce qu'on éthérifie des dérivés d'acide hydroxyphénylacétique de formule 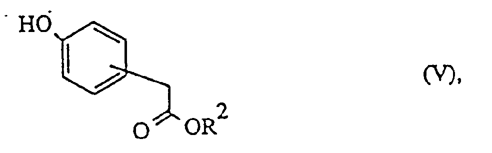 dans laquelle
R² est un groupe (+)- ou (-)-menthyle, (+)- ou (-)-bornyle, (+)- ou (-)-isobornyle ou (-)-8-phénylmenthyle
avec des halogénométhylquinoléines de formule générale VI 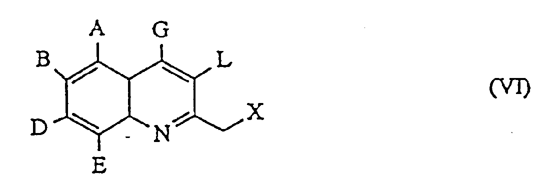 dans laquelle
A, B, D, E, G et L ont la définition indiquée ci-dessus
et
X représente un halogène,
dans des solvants inertes, le cas échéant en présence d'une base et/ou d'une substance auxiliaire, ou par catalyse avec transfert de phase.
